(19) **Europäisches Patentamt / European Patent Office / Office européen des brevets**

(11) **EP 1 989 990 B1**

(12) **EUROPEAN PATENT SPECIFICATION**

(45) Date of publication and mention
of the grant of the patent:
**04.06.2014 Bulletin 2014/23**

(51) Int Cl.:
*A61B 1/00* *(2006.01)*     *A61B 17/00* *(2006.01)*
*A61B 1/313* *(2006.01)*

(21) Application number: **06714780.1**

(22) Date of filing: **27.02.2006**

(86) International application number:
**PCT/JP2006/303643**

(87) International publication number:
**WO 2007/097034 (30.08.2007 Gazette 2007/35)**

(54) **ENDOSCOPIC SURGERY TOOL**

ENDOSKOPISCHES CHIRURGIEGERÄT

INSTRUMENT CHIRURGICALE ENDOSCOPIQUE

(84) Designated Contracting States:
**DE FR GB IE**

(43) Date of publication of application:
**12.11.2008 Bulletin 2008/46**

(73) Proprietor: **OLYMPUS MEDICAL SYSTEMS CORP.
Tokyo 151-0072 (JP)**

(72) Inventors:
• **MINOSAWA, Ryo
Tsukui-gun, Kanagawa-ken 220-0112 (JP)**
• **DEJIMA, Takumi
Mitaka-shi, Tokyo 181-0013 (JP)**
• **MIYAMOTO, Manabu
Shibuya-ku, Tokyo (JP)**
• **MATSUNO, Kiyotaka
Sagamihara-shi, Kanagawa-ken 229-1124 (JP)**

(74) Representative: **von Hellfeld, Axel
Wuesthoff & Wuesthoff
Patent- und Rechtsanwälte
Schweigerstrasse 2
81541 München (DE)**

(56) References cited:
JP-A- 05 337 118     US-A- 5 572 999
US-A- 5 797 835     US-A- 5 836 869
US-B2- 6 648 816

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

*Technical Field*

The present invention relates to an endoscopic surgery tool that is used when treatment is performed inside the body cavity of a patient.

*Background Art*

Conventionally, it has been the practice when a surgical operation is performed while using an endoscope to open a dedicated port in the body wall in order to insert a rigid endoscope that is used to observe the body cavity interior. In a surgical operation performed while using an endoscope, because a rigid endoscope is inserted through a single fixed port, altering the field of view in the body cavity interior has been achieved by tilting the rigid endoscope using the port as a support point so that the observable field of view has been limited. Here, in order to observe multi-directionally, because it is necessary to insert into the body cavity interior the same number of endoscopes as the number of required viewing points, the invasiveness towards the patient is increased. Moreover, a scopist is required to operate the endoscope during an operation and the problem has occurred that it is sometimes difficult for a surgeon to convey their wishes to the scopist. In addition, because observation images provided by an endoscope during an operation are intended principally to be images for the surgeon, it is difficult for an assistant to find the proper orientation.

One conventional technology designed to solve these problems is an instrument in which an image pickup device is fixed onto a guide shaft through which a treatment tool can be inserted, with the image pickup device being offset from the guide shaft (see, for example, Patent document 1). If the guide shaft is inserted into a body cavity interior via a trocar, then a dedicated port for the endoscope is not required. Because the image pickup device is offset from the guide shaft, observation from a different angle from the axial direction of the guide shaft becomes possible. Furthermore, by tilting the guide shaft, it is possible to alter the field of observation of the image pickup device. Because this type of instrument can be used in all of the treatment ports that are used in an operation, a plurality of viewing points can be obtained without burdening the patient with any invasiveness other than the treatment ports. Moreover, because a visual field is obtained specifically for the assistant, superior hand-eye coordination can be obtained.
Patent document 1: United States Patent No. 6,648,816 Specification

*Disclosure of the Invention*

*Problems to be Solved by the Invention*

In this manner, in a conventional instrument, the visual field of an image pickup device always faces towards the distal end of the treatment tool, however, in an actual operation, when an intricate task having a small visual field such as suturing is being performed, if forceps which have been inserted into the guide shaft during this task in order to perform treatment are tilted, then the image pickup device which is fixed to the guide shaft is also tilted in the same way. Because the observation image also becomes tilted if the image pickup device is tilted, the task becomes difficult to perform. In addition, while the distal end portion of the treatment tool remains in the treatment location, it is not possible to confirm another subject position existing outside the observation visual field, for example, to confirm the traction state of an internal organ outside the visual field. Moreover, in the instrument disclosed in Patent document 1, the angle between the axis of the treatment tool insertion direction and an axis showing the direction of the image pickup visual field of the image pickup device is determined in advance, and cannot be altered while the task is being performed. Because of this, depending on the circumstances of the task, the task location may be located in a dead angle, and it may be difficult to continuously provide the optimum visual field.

The present invention was conceived in view of the above described circumstances, and it is a principal object thereof to enable the optimum visual field to be obtained during a treatment.

*Means for Solving the Problem*

This object is addressed by an endoscopic surgery tool according to claim 1.

A first aspect of the present invention is an endoscopic surgery tool in which an image pickup device is provided on a hollow guide component that penetrates a body wall of a living body and guides a surgical tool into a body cavity, that includes: a detection device that detects an amount of change in the tilt of the guide component when the guide component is tilted relative to the body wall using the portion passing through the body wall as a support point; and a correction device that calculates from the tilt change amount an amount of movement of the image pickup device that moves in conjunction with the tilting of the guide component, and makes corrections in order that an observation image from the image pickup device before the guide component was tilted is maintained after it has been tilted.

This endoscopic surgery tool is constructed such that an image pickup device is able to move freely relatively to a guide component. Accordingly, even after the guide component has been tilted under the control of the correction device, it is possible to maintain the observation image from before the tilting.

A second aspect of the present invention is the endoscopic surgery tool according to the first aspect of the present invention in which there are provided: a calculation unit that calculates an amount of movement of the image pickup device such that the position as well as the direction of the optical axis of the image pickup device before the guide component is tilted substantially coincide after it has been tilted; and a drive device that causes the image pickup device to move in accordance with the result of the calculation by the calculation unit.

In this endoscopic surgery tool, for example, the position and amount of turn of the image pickup device are calculated as an amount of movement by a calculation unit, and the image pickup device is then moved by the drive device.

A third aspect of the present invention is the endoscopic surgery tool according to the second aspect of the present invention in which the drive device has a connecting component that has one end portion that is pivotably mounted on the guide component and has another end portion that is pivotably mounted on the image pickup device, and in which the length from the one end portion to the other end portion can be expanded or contracted.

In this endoscopic surgery tool, the position of the image pickup device is adjusted by pivoting the connecting component relative to the guide component, and by also changing the length of the connecting component. Furthermore, the direction of the visual field of the image pickup device is adjusted by causing the image pickup device to pivot relative to the connecting component.

A fourth aspect of the present invention is the endoscopic surgery tool according to the second aspect of the present invention in which the drive device has a connecting component that has one end portion that is pivotably mounted on the guide component and has another end portion that is fixed to the image pickup device, and in which the length from the one end portion to the other end portion can be expanded or contracted, and in which the correction device has an image processing unit that changes the display range of images acquired by the image pickup device such that they remain substantially constant before and after the position of the image pickup device changes.

In this endoscopic surgery tool, the position of the image pickup device is adjusted by pivoting the connecting component relative to the guide component, and by also changing the length of the connecting component. Furthermore, image processing is performed on images acquired from the image pickup device so as to alter the display range thereof so that an observation image that is displayed on the display unit is maintained before and after the guide component is tilted.

A fifth aspect of the present invention is the endoscopic surgery tool according to the first aspect of the present invention in which there are provided: a calculation unit that calculates an amount of movement of the image pickup device such that the direction of the optical axis of the image pickup device remains substantially constant before and after the guide component is tilted; and a drive device that causes the image pickup device to move in accordance with the result of the calculation by the calculation unit, and that also changes the viewing angle of the image pickup device.

In this endoscopic surgery tool, the position of the image pickup device is calculated by the calculation unit, and the image pickup device is then moved by the drive device. Furthermore, the drive device is driven and the visual field of the image pickup device is changed so as to be kept constant before and after the guide component is tilted.

A sixth aspect of the present invention is the endoscopic surgery tool according to the fifth aspect of the present invention in which the drive device has a connecting component that has one end portion that is fixed to the guide component and has another end portion that is pivotably mounted on the image pickup device, and in which the length from the one end portion to the other end portion can be expanded or contracted, and in which the correction device is constructed so as to drive the connecting component such that the optical axis of the image pickup device remains substantially constant before and after the image pickup device is moved in conjunction with the tilting of the guide component, and is constructed such that an observation image is maintained before and after the movement of the image pickup device by altering the viewing angle of the image pickup device.

In this endoscopic surgery tool, the length of the connecting component is changed resulting in an adjustment to the position of the image pickup device. Furthermore, the direction of the visual field of the image pickup device is adjusted by causing the image pickup device to pivot relative to the connecting component, and by altering the viewing angle thereof.

A seventh aspect of the present invention is the endoscopic surgery tool according to the fifth aspect of the present invention in which the drive device has a connecting component that has one end portion that is fixed to the guide component and has another end portion that is fixed to the image pickup device, and in which the length from the one end portion to the other end portion can be expanded or contracted, and in which the correction device is constructed so as to drive the connecting component such that the optical axis of the image pickup device remains substantially constant before and after the image pickup device is moved in conjunction with the tilting of the guide component, and is constructed such that an observation image is maintained before and after the movement of the image pickup device by altering the viewing angle of the image pickup device and by also altering the display range of images acquired by the image pickup device.

In this endoscopic surgery tool, the length of the connecting component is changed so as to adjust the position of the image pickup device. The direction of the visual field of the image pickup device is adjusted by altering the viewing angle of the image pickup device. Furthermore, image processing is performed on images acquired from the image pickup device resulting in an alteration to the display range thereof so that an observation image that is displayed on the display unit is maintained before and after the guide component is tilted.

An eighth aspect of the present invention is the endoscopic surgery tool according to any one of the second through seventh aspects of the present invention in which the detection device is an acceleration sensor.

In this endoscopic surgery tool, because attention was given to the fact that the output from the acceleration sensor changes depending on the tilt change amount, the position of the image pickup device is controlled in accordance with the amount of change in the output from the acceleration sensor.

A ninth aspect of the present invention is the endoscopic surgery tool according to any one of the second through seventh aspects of the present invention in which the detection device is constructed so as to make a calculation using the amount of movement of an arbitrary point within the visual field of the image pickup device.

In this endoscopic surgery tool, because a predetermined location in an image acquired by the image pickup device moves if the guide component is tilted, the amount of change in the tilt of the guide component is calculated by calculating the amount of this movement.

*Effects of the Invention*

According to the present invention, because observation images that are acquired by an image pickup device and are provided to a surgeon are kept constant by processing performed by a correction device before and after a guide component is tilted, it is possible to prevent an observation image being blurred while a task is being performed. Furthermore, it is difficult for a location where a task is being performed to be hidden in a dead angle. Because of these advantages, performing a task inside a body cavity is made easier.

*Brief Description of the Drawings*

FIG. 1 shows the schematic structure of an endoscopic surgery tool according to a first embodiment.
FIG. 2 is a view to illustrate a procedure to calculate an amount of movement of an image pickup device in order to make the positions of the image pickup device substantially coincide before and after a guide component is tilted.
FIG. 3 is a view illustrating FIG 2 represented by a localized coordinate system.
FIG. 4 is a view illustrating the state when a point within the visual field of an image pickup device is moved by tilting a guide component.
FIG. 5 is a view to illustrate a procedure to calculate an amount of turn by which an image pickup device is turned such that the direction of the optical axis of the image pickup device remains substantially constant when a guide component is tilted.
FIG. 6 shows the schematic structure of an endoscopic surgery tool according to a second embodiment.
FIG. 7 is a view showing a state in which observation images are made to substantially coincide after a guide component is tilted.
FIG. 8 shows the schematic structure of an endoscopic surgery tool according to a third embodiment.
FIG. 9 is a view to illustrate a procedure to calculate an amount of movement of an image pickup device before and after a guide component is tilted.
FIG. 10 is a view to illustrate a procedure to adjust the visual field and focus of an image pickup device such that the direction of the optical axis of the image pickup device is made to remain substantially constant when a guide component is tilted.
FIG. 11 is an example of an observation image display when a hard mirror is also employed.
FIG. 12 is a view showing a structure in which an acceleration sensor is used as a detection device.

*Description of the Reference Numerals*

| | |
|---|---|
| 1 | Endoscopic surgery tool |
| 3 | Processor (Correction device, Detection device) |
| 3a | Signal processing device (Operating unit) |

| 3e | Image processing unit |
| 4 | Display unit |
| 10 | Hollow shaft (Guide component) |
| 11 | Connecting component (Drive device) |
| 14, 14a, 14b | Image pickup device |
| 40 | Optical zoom mechanism (Drive device) |

*Best Mode for Carrying Out the Invention*

Embodiments of the present invention will be described hereinafter using drawings. Note that the same symbols are allocated to identical component elements in the respective embodiments and any duplicate description thereof is omitted.

*(First embodiment)*

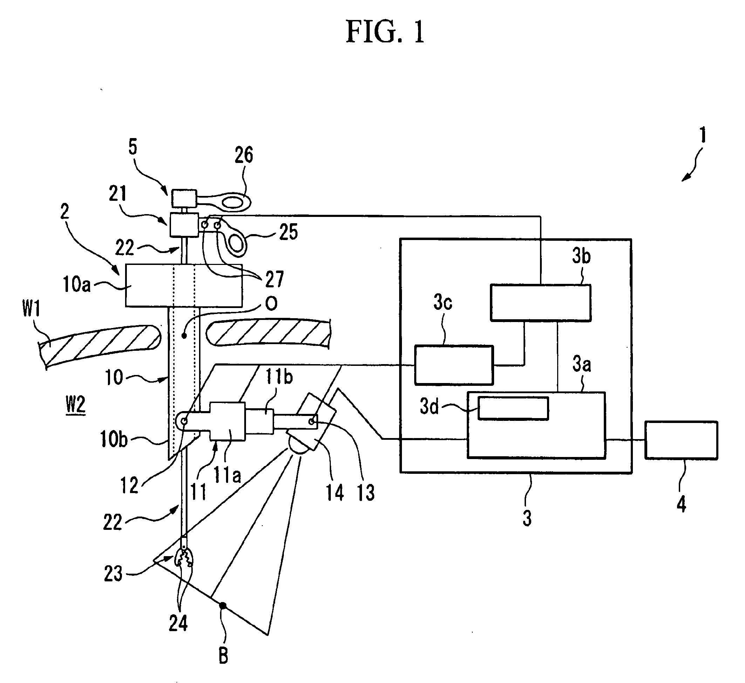

FIG. 1 shows an overall view of the present invention. An endoscopic surgery tool 1 includes a trocar 2 for endoscopic surgery, a correction device in the form of a processor 3, a display unit 4, and an operating tool in the form of a treatment tool 5. Note that the processor 3 also functions as a detection device to detect the angle of inclination of the trocar 2 for endoscopic surgery as is described below.

The trocar 2 for endoscopic surgery is a guide component which has a hollow shaft 10 that penetrates a body wall W1. A base end portion 10a of the hollow shaft 10 has an enlarged diameter. The base end portion 10a is the portion that is used outside the body, and is the closest end as seen from the surgeon's position. A connecting component 11 is attached by a connecting pin 12 to a distal end portion (i.e., to the far end as seen from the surgeon's position) 10b of the hollow shaft 10 which is inserted into the body interior. The connecting component 11 is able to be pivoted freely around the connecting pin 12 by means of a motor (not shown). The connecting component 11 is a nested structure having a main body portion 11a which is on the connecting pin 12 side and a slider 11b which is slidably inserted into the main body portion 11a. The connecting component 11 is a driving device in which the length (i.e., the amount of expansion or contraction) of the connecting component 11 can be changed by controlling the amount that the slider 11b protrudes from the main body portion 11a. For example, a motor (not shown) can be used to control the amount of expansion or contraction of the connecting component 11. An observation device in the form of an image pickup device 14 is pivotably mounted by means of a connecting pin 13 onto a distal end portion of the slider 11b which is protruding from the main body portion 11a.

The image pickup device 14 has image pickup elements such as, for example, CCD (Charge Coupled Devices) or CMOS (Complimentary Metal Oxide Semiconductor), and output signals therefrom are input by cable or wireless into the processor 3. It is also possible to further provide a light emitting diode or a light guide cable as an illumination device in the image pickup device 14. The image pickup device 14 is able to be freely pivoted around the connecting pin 13 by a motor (not shown). Note that it is also possible to employ a structure in which, instead of the connecting component 11 pivoting around the connecting pin 12, the connecting component 11 is curved relative to the hollow shaft 10.

The processor 3 has a calculation unit in the form of a signal processing device 3a that processes signals from the image pickup device 14, a control circuit 3b that calculates the amount of protrusion of the connecting component 11, the amount of rotation of the connecting component 11 around the axis of the connecting pin 12, and the amount of rotation of the image pickup device 14 around the axis of the connecting pin 13, and a drive device in the form of a drive circuit 3c that actually drives the motor or the like that causes the connecting component 11 to pivot. The signal processing device 3a has a tilt detection unit 3d that, when the hollow shaft 10 is tilted taking substantially the center of a portion passing through the body wall W1 as a support point O, detects the amount of tilt of the hollow shaft 10 from the amount of movement of an arbitrary point B within the observation visual field. The control circuit 3b uses the amount of tilt calculated by the tilt detection unit 3d to calculate the amount of turn and the amount of expansion or contraction of the connecting component 11 in order for the image pickup device 14 to remain in substantially the same position before and after the tilting of the hollow shaft 10. Furthermore, after calculating the amount of turn of the image pickup device 14 that picks up an image of an arbitrary point from substantially the same direction, the control circuit 3b transmits a signal to the drive circuit 3c. The drive circuit 3c causes the connecting component 11 to turn and expand or contract, and also causes the image pickup device 14 to turn.

The treatment tool 5 has a rigid insertion portion 22 that extends from an operating portion 21 which is operated by a surgeon, and is replaceably inserted into the trocar 2 for endoscopic surgery. A treatment portion 23 is provided at a distal end portion of the insertion portion 22. In the treatment tool 5 of this embodiment, the treatment portion 23 is formed by gripping forceps that is provided with a pair of gripping components 24 that are able to open and close freely. The treatment tool 5 is not limited to gripping forceps, and may be another type of treatment tool. The operating portion 21 has handles 25 and 26 that cause the pair of gripping components 24 to open and close. Furthermore, an operating device 27 is provided that operates the motor which causes the connecting component 11 to turn and expand or contract, and operates the motor that causes the image pickup device 14 to turn, and also alters the viewing angle of the image pickup device 14, and is thereby able to freely manipulate the field of view of the image pickup device 14. Examples of the operating device 27 include buttons, switches, and the like.

A description of the operation of this embodiment will now be given.

When a surgeon inserts the trocar 2 for endoscopic surgery into a body cavity W2, the surgeon operates the operating device 27 so that the hollow shaft 10, the connecting component 11, and the image pickup device 14 are placed on the same axis. At this time, the control circuit 3b receives signals from the operating device 27 and calculates pivot angles that will allow the hollow shaft 10, the connecting component 11, and the image pickup device 14 to be lined up on the same axis. The motor is then driven by the drive circuit 3c. As a result of the trocar 2 for endoscopic surgery becoming substantially rectilinear, it is able to be inserted into a body without the diameter of the treatment port being widened much more than is the case conventionally.

Images inside the body interior W2 are acquired by the image pickup device 14 of the trocar 2 for endoscopic surgery. Because of this, a rigid scope insertion port is not required. When a plurality of treatment tools are to be used, because a plurality of treatment ports are used, if a trocar 2 for endoscopic surgery is inserted into each one of these treatment ports, observation from multiple viewpoints becomes possible. Furthermore, if a trocar 2 for endoscopic surgery is inserted into a port for an assistant, then a dedicated viewpoint for the assistant is obtained and the assistant is able to achieve superior hand-eye coordination. Note that if a plurality of image pickup devices 14 are provided for a single trocar 2 for endoscopic surgery, then observation over a wider range and from multiple viewpoints becomes possible.

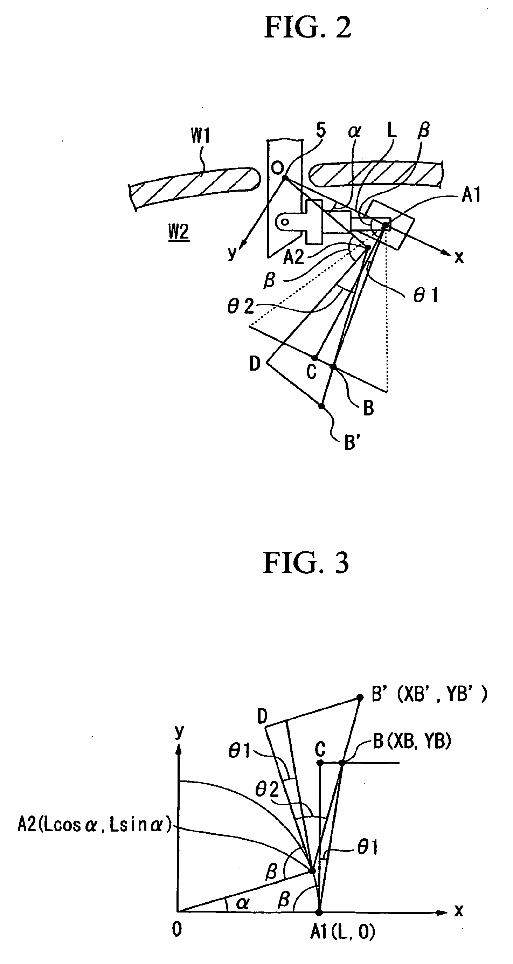

If the hollow shaft 10 tilts the body wall W1 around the support point O, the tilt detection unit 3d calculates the amount of tilt of the hollow shaft 10 from the amount of movement of an arbitrary point B in the observation visual field. A description will now be given of the procedure to calculate a tilt amount. FIG. 2 and FIG. 3 are explanatory views showing in typical form a tilt amount calculation method. In FIG. 2, a position of the image pickup device 14 prior to the hollow shaft 10 being tilted is taken as A1, and the focus position on the optical axis at this time is taken as C. if the hollow shaft 10 is tilted by an angle $\alpha$ (= $\angle$A1OA2) and the connecting component 11 is neither turned, expanded, nor contracted and the image pickup device 14 is not turned, then the position of the image pickup device 14 moves along an arc centered on the support point O. The position of the image pickup device 14 at this time is taken as A2, and the focus position on the optical axis is taken as D. In FIG. 3, a localized coordinate system is employed in which a vector OA1 is plotted on an X axis, O is the point of origin, and a Y axis is an axis which is perpendicular to the X axis.

An angle $\beta$ between a line segment connecting the support point O to the position A1 of the image pickup device 14 and a line segment connecting the position A1 with the focus position C on the optical axis of the image pickup device 14 is a known amount, and does not change before and after the image pickup device 14 is tilted. Namely, $\beta$ = $\angle$OA1C = $\angle$ OA2D.

Here, at the arbitrary point B within the observation visual field (i.e., within the image pickup range), the viewing angle from the optical axis before the image pickup device 14 is tilted is taken as $\theta1$ (=$\angle$BA1C). The viewing angle from the optical axis at the arbitrary point B (i.e., at the point B' in FIG 3) after the image pickup device 14 has been tilted is taken as $\theta2$ (=$\angle$B'A1C).

If the coordinates of the arbitrary point B are taken as (XB, YB), then YB which is the Y coordinate of the point B can be calculated from each of the two types of formula given below.

[Formula 1]

$$YB = XB \times \tan(S + \alpha) + L \times \sin\alpha - L \times \tan(S + \alpha) \times \cos\alpha$$

[Formula 2]

$$YB = XB \times \tan T - L \times T$$

wherein $T = \pi - \beta - \theta1$ ; and $S = \pi - \beta - \theta2$.

Here, if the focus position C of the image pickup device 14 is detected, because the coordinates of point B (XB, YB) can be calculated by means of image processing or the like using the coordinates of the focus position C and the direction and distance from the focus position C, they are treated as known values. Furthermore, the viewing angle $\theta1$ of the arbitrary point B from the optical axis before the image pickup device 14 is tilted is also a known value. Accordingly, if the viewing angle $\theta2$ is supplied, the amount of change $\alpha$ in the tilt of the hollow shaft 10 can be obtained from the above two formulas.

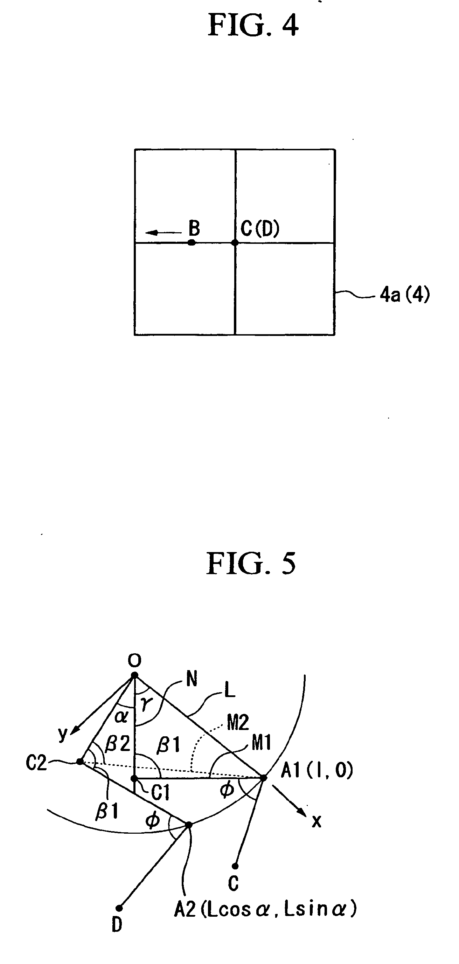

Namely, as is shown by the arrow in FIG 4, if the hollow shaft 10 is tilted, because an image which corresponds to the point B moves on a display screen 4a of the display unit 4, using the direction of movement and the amount of movement of the arbitrary point B which is displayed on the screen, the amount of increase in the viewing angle from the optical axis is detected, thereby enabling the control circuit 3b to calculate θ2. In addition, α can be found using the above described relational formulae and the tilt amount consequently determined. Accordingly, it is possible to determine the tilt change amount α from the amount of movement and direction of movement of the arbitrary point B.

Once the tilt detection unit 3d has calculated the tilt change amount α, based on this tilt change amount α, the control circuit 3b calculates the amount of turn and the amount of expansion or contraction of the connecting component 11 such that the position of the image pickup device 14 remains substantially constant before and after the tilting of the hollow shaft 10. Furthermore, the control circuit 3b also calculates the amount of turn of the image pickup device 14 that will enable it to obtain an image of the point B from substantially the same direction, and sends the calculation results to the drive circuit 3c. The drive circuit 3c causes the connecting component 11 to turn and expand or contract in accordance with the calculation result, and thereby causes the image pickup device 14 to turn.

A method of calculating the amount of turn and the amount of expansion or contraction of the connecting component 11, and of calculating the amount of turn of the image pickup device 14 is shown in typical form in FIG 5. FIG 5 employs the same local coordinate system as that used in FIG. 3. The position of the connecting pin 12 (i.e., the turn portion) of the connecting component 11 prior to the tilting of the hollow shaft 10 is taken as C1, while the position thereof after the tilting is taken as C2. Note that the distance from the support point O to the position C1 is a known amount N. An angle β between a line segment connecting the support point O to the position C 1 (or the position C2) and a line segment connecting the position C 1 (or the position C2) to the position A1 (or the position A2) of the image pickup device 14 is a known amount, namely, β = ∠OC1A1 = ∠OC2A2. The direction φ (wherein φ = ∠C1A1C = ∠C2A2D) of the visual field relative to the connecting component 11 is a known amount and is a value which is controlled by the processor 3 after the hollow shaft 10 has been inserted into the body cavity W2 but before it has been tilted. In the same way, an angle γ ( wherein γ = ∠A1OC1) between a line segment connecting the support point O and the position A1 before the image pickup device 14 is tilted, and the axis of the hollow shaft 10 is a known amount before the hollow shaft 10 is tilted. Furthermore, a length M1 (i.e., the length of the line segment C1A1) of the connecting component 11 before the trocar 2 for endoscopic surgery is tilted is a known amount before the hollow shaft 10 is tilted.

If the connecting component 11 is moved such that the visual field remains substantially constant before and after the hollow shaft 10 is tilted, then the length and turning angle of the connecting component 11 changes. The length of the connecting component 11 at this time is taken as M2. In the same way, an angle (i.e., the turning angle) between the hollow shaft 10 and the connecting component 11 is taken as β2. In this case M2 and β2 are expressed in the manner described below.

[Formula 3]

$$M2 \doteq \sqrt{(N \times cos\ (\alpha + \gamma) - L)^2 + (N \times sin(\alpha + \gamma))^2}$$

[Formula 4]

$$\beta2 = arctan\ [1/(tan\ (\alpha + \gamma))] + arctan\ (|(L - N \times cos\ (\alpha + \gamma))/(N \times sin\ (\alpha + \gamma))|)$$

The amount of turn of the connecting component 11 is calculated as β1 - β2. The amount of expansion or contraction of the connecting component 11 is calculated as M2 - M1. The amount of turn of the image pickup device 14 is calculated as α - β1 + β2. These values are obtained from a relational formula which takes the tilt change amount α of the hollow shaft 10 as a change amount. If the tilt change amount α of the hollow shaft 10 is known, then it is possible to control the position of the image pickup device 14 such that it obtains an image of an arbitrary point from substantially the same direction in substantially the same position and displays it on the display unit 4.

Accordingly, even when the hollow shaft 10 is tilted with the body wall W1 used as a support point O, the image pickup device 14 remains substantially unaffected and maintains a state observation in which an observation image remains unchanged (i.e., a state in which observation images substantially coincide). Moreover, because it is possible to independently alter the visual field by means of the operating device 27 of the treatment tool 5, a user is able to perform an operation to alter the visual field without having to remove their hand from the treatment tool 5.

In the present embodiment, because the image pickup device 14 is provided such that it is able to pivot freely in a trocar (i.e., the hollow shaft 10), because the image pickup device 14 is positioned substantially parallel with the insertion axis of the hollow shaft 10 when it is inserted into the body cavity W2, it is able to be inserted into the body cavity W2 without the diameter of the treatment port being substantially increased in width. Furthermore, because an endoscope port which has hitherto been considered indispensable is no longer required, the invasiveness to the patient is reduced and rapid recovery can be achieved. By providing the image pickup device 14 in the hollow shaft 10, it is possible to obtain a plurality of viewing points inside the body cavity W2 without increasing the invasiveness beyond that imposed by the treatment port. Dead angles are decreased and it becomes easy to ascertain the shape of a subject portion. Because safe treatment can be performed, operation times can be shortened. Because it becomes possible to provide a dedicated viewing point for an assistant, the assistant is easily able to obtain superior hand-eye coordination. As a result, cooperation between a surgeon and an assistant is made easier and operation times can be shortened.

Furthermore, if this endoscopic surgery tool 1 is used, there is no limit to the range over which observation is possible through a treatment port. Because the operating device 27 is provided in the endoscopic surgery tool 1, a user of the trocar 2 for endoscopic surgery is able to freely alter the observation visual field by altering the viewing point and visual field of the image pickup device 14 without taking their hand away from the operation of the treatment tool 5. Accordingly, cooperation with a scopist is no longer required, as it is conventionally, and the most appropriate visual field for treatment can be obtained. It is possible to shorten operation times and also perform safe treatment. Because it becomes possible to alter a visual field independently of the movement of a treatment tool by operating the operating device 27, even, for example, in a state in which it is not possible to move the distal end portion of the treatment tool 5, it becomes possible to observe a range outside that of the treatment portion so that even safer treatment is made possible.

Moreover, even when the trocar 2 for endoscopic surgery is tilted using the body wall W1 as a support point, there is no shifting of an observation image. When intricate treatment over a small range such as suturing is being performed, operability is improved, and it is possible to achieve both safe treatment and a reduction in operation time.

*(Second embodiment)*

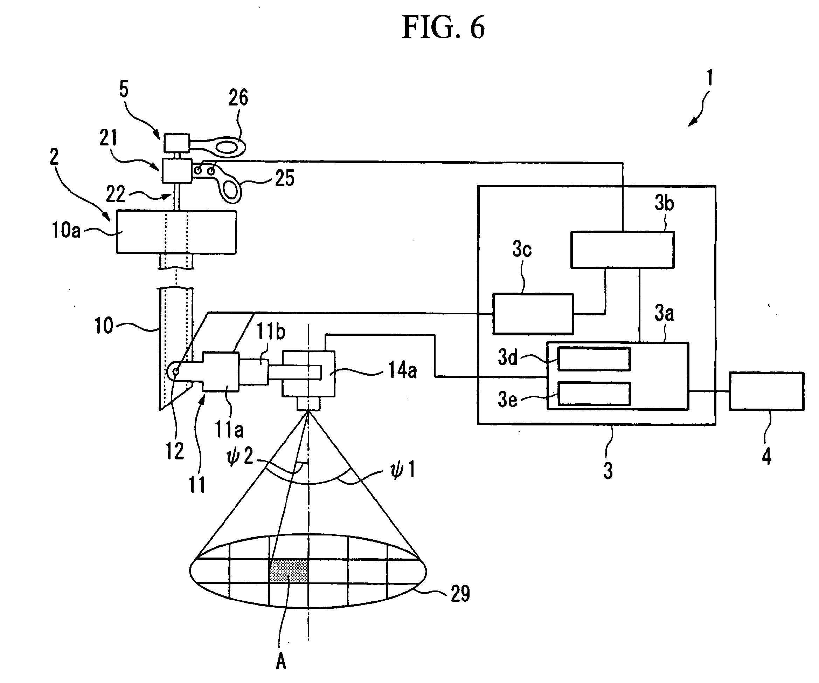

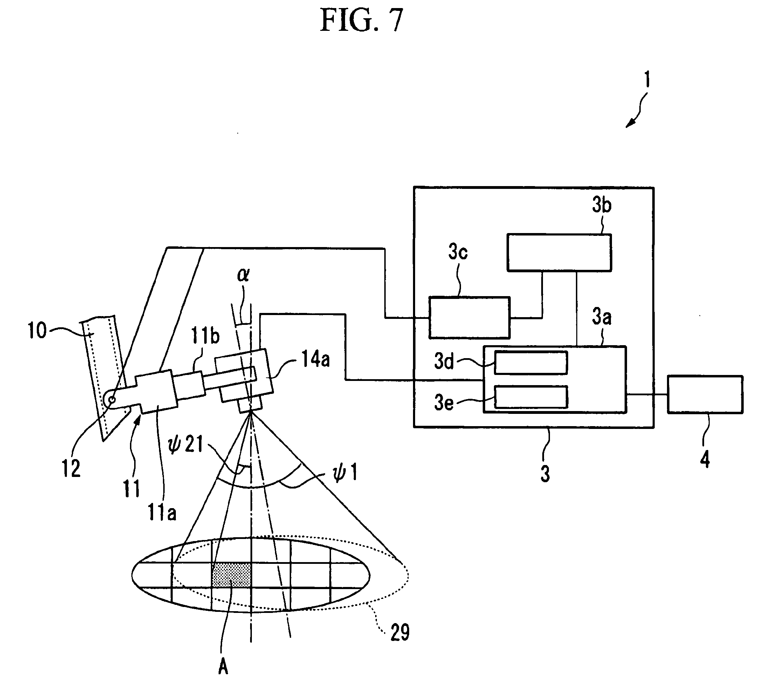

FIG. 6 and FIG. 7 show the structure of the device of the present embodiment.

As is shown in FIG. 6, the endoscopic surgery tool 1 has an image pickup device 14a that is fixed to a connecting component 11 that forms a drive device. The image pickup device 14a has an optical system having an alterable viewing angle, and has image pickup elements such as CCD (Charge Coupled Devices) or CMOS (Complimentary Metal Oxide Semiconductor), and output signals therefrom are input by cable or wireless into the processor 3. The viewing angle (i.e., the screen angle) $\phi 1$ of the image pickup device 14a is large enough to obtain a viewing angle (i.e., screen angle) $\phi 2$ that corresponds to an image pickup range 29. The processor 3 has an image processing unit 3e and is constructed so as to be able to arbitrarily alter the display range of images acquired by the image pickup device 14a by means of image processing (i.e., using a digital zoom function). Note that this altering of the display range can be performed by the operating device 27 (see FIG 1).

A description will be given of the operation of this embodiment.

Firstly, the maximum image pickup range 29 when the image pickup device 14a is used at the wide viewing angle $\phi 1$ is displayed on the display unit 4. As is shown in FIG. 7, when the trocar 2 for endoscopic surgery is tilted by a tilt change amount $\alpha$ around the support point O, then if a surgeon wishes to display an arbitrary subject display portion A within the image pickup range 29, the surgeon alters the line of sight of the observation visual field and alters the zoom using the operating device 27 (see FIG 6) while observing the area displayed on the display unit 4. As a result, the display range of the images acquired by the image pickup device 14a and displayed on the display unit 4 changes to an image corresponding to a screen angle $\phi 21$. Namely, the same result is obtained as when the viewing angle of the image pickup device 14a is changed, and an image of the subject display portion A is displayed on the display unit 4.

Accordingly, even when the hollow shaft 10 is tilted with the body wall W1 used as a support point O, the image pickup device 14a remains substantially unaffected and maintains a state observation in which an observation image remains unchanged (i.e., a state in which observation images substantially coincide). Moreover, because it is possible to independently alter the visual field by means of the operating device 27 of the treatment tool 5, a user is able to perform an operation to alter the visual field without having to remove their hand from the treatment tool 5.

In the present embodiment, because it is possible by adjusting the display range of the image pickup device 14a to obtain a display screen that remains substantially unchanged before and after the trocar 2 for endoscopic surgery is tilted, no mechanical correction device is required. Accordingly, the structure of the device can be simplified. The remaining effects are the same as those obtained in the first embodiment.

Here, when the trocar 2 for endoscopic surgery is moved two-dimensionally within a plane whose domain is defined by the axis of the connecting component 11 and the axis of the hollow shaft 10, the optical axis of the image pickup device 14a remains constant. In contrast to this, if the trocar 2 for endoscopic surgery is moved three-dimensionally, then by providing a bending and contracting function in the connecting component 11 and also providing a function of pivoting the image pickup device 14a, if the position of the image pickup device 14a is adjusted by the processing of the processor 3, the optical axis of the image pickup device 14a can be kept constant.

Note that in the trocar 2 for endoscopic surgery according to the present embodiment, a structure is employed in which a surgeon manually adjusts the display range of the image pickup device 14a, however, it is also possible for the display range of the image pickup device 14a to be held constant automatically by the processing of the processor 3. In this case, the control circuit 3b calculates the amount of protrusion of the connecting component 11, the amount of rotation of the connecting component 11, and the amount of rotation of the image pickup device 14a and the like, and then creates a drive signal to cause a motor or the like to be driven. It then moves the position of the image pickup device 14a in the same way as in the first embodiment.

Moreover, it is also possible to employ a structure in which it is possible to switch between manually adjusting the display range of the image pickup device 14a, and adjusting the image pickup position of the image pickup device 14a by means of automatic processing. If a switch or button that makes this switch is provided in a position which is easily accessible to a surgeon, then it is possible to further improve the treatment efficiency.

(*Third embodiment*)

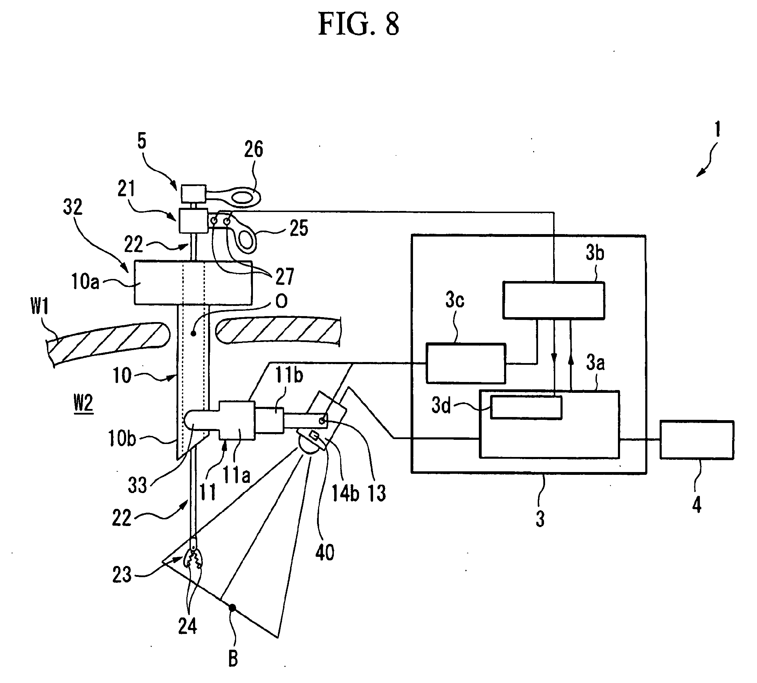

FIG. 8 through FIG. 10 show the structure of an endoscopic system of the present embodiment.

As is shown in the view of the overall structure in FIG. 8, the endoscopic surgery tool 1 has a trocar 32 for endoscopic surgery, a process and 3, a display unit 4, and a treatment tool 5.

The trocar 32 for endoscopic surgery has a hollow shaft 10. A base end portion 10a of the hollow shaft 10 has an enlarged diameter. The base end portion 10a is the portion that is used outside the body, and is the closest end as seen from the surgeon's position. A connecting component 11 that forms a driving device is attached to a distal end portion (i.e., to the far end as seen from the surgeon's position) which is inserted into the body interior. The connecting component 11 has a nested structure having a main body portion 11a whose base end portion 33 is fixed to the hollow shaft 10, and a slider 11b which is slidably inserted into the main body portion 11a. The length (i.e., the amount of expansion or contraction) of the connecting component 11 can be changed by controlling the amount that the slider 11b protrudes from the main body portion 11a. For example, a motor (not shown) can be used to control the amount of expansion or contraction of the connecting component 11. An image pickup device 14b is mounted by means of a connecting pin 13 onto a distal end portion of the slider 11b which is protruding from the main body portion 11a. The image pickup device 14b can be freely pivoted around the connecting pin 13 by a motor (not shown), and has an optical zoom mechanism 40 (i.e., a driving device) that alters the viewing angle in an internal optical system.

The processor 3 has a calculation unit in the form of a signal processing device 3a that processes signals from the image pickup device 14, a control circuit 3b that calculates drive amounts and the like, and a drive device in the form of a drive circuit 3c that actually drives the motor or the like. The signal processing device 3a has a tilt detection unit 3d that, when the hollow shaft 10 is tilted taking substantially the center of a portion passing through the body wall W1 as a support point O, detects the amount of tilt of the hollow shaft 10 from the amount of movement of an arbitrary point B within the observation visual field. The control circuit 3b uses the amount of tilt calculated by the tilt detection unit 3d to calculate the amount of expansion or contraction of the connecting component 11 in order for the image pickup device 14b to remain on substantially the same optical axis before and after the tilting of the hollow shaft 10. Furthermore, the control circuit 3b calculates a viewing angle that enables an observation image to be maintained. The drive circuit 3c causes the connecting component 11 to turn and expand or contract, and also causes the image pickup device 14 to turn. The drive circuit 3c receives information from the control circuit 3b and causes the connecting component 11 to expand or contract, and thereby causes the image pickup device 14b to turn, and in addition alters the viewing angle by driving the optical zoom mechanism 40.

An operation of this embodiment will now be described.

A surgeon operates the operating device 27 so as to place the hollow shaft 10, the connecting component 11, and the image pickup device 14b on the same axis. After this, the trocar 2 for endoscopic surgery is inserted into the body cavity W2. After this insertion, the image pickup device 14b is controlled by the processor 3 so as to be placed in a predetermined position.

If the hollow shaft 10 is then tilted using the body wall W1 as a support point O, using the same method as that used in the first embodiment, the tilt detection unit 3d calculates the tilt change amount $\alpha$ of the hollow shaft 10 from the amount of movement of the arbitrary point B.

Next, the control circuit 3b calculates from this tilt change amount $\alpha$ the amount of expansion or contraction of the connecting component 11 such that it substantially coincides with the position of the optical axis before the tilting of the image pickup device 14b. Furthermore, the control circuit 3b also calculates the amount of turn of the image pickup device 14b that will enable it to obtain an image of the point B from substantially the same direction. These calculation results are sent to the drive circuit 3c. The drive circuit 3c drives the hollow shaft 10 to expand or contract and drives the connecting component 11 to turn. Moreover, the control circuit 3b calculates a viewing angle that enables the observation image to be maintained, and the viewing angle is then altered by the signal processing device 3a.

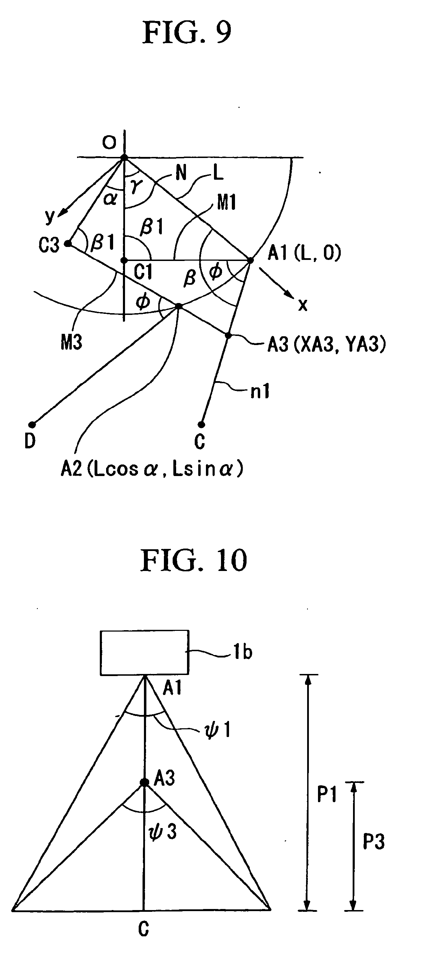

FIG 9 is an explanatory view showing a method of calculating the amount of expansion or contraction of the connecting component 11, and of calculating the amount of turn of the image pickup device 14. FIG 9 employs the same local coordinate system as that used in FIG 5. The position of the base end portion 33 of the connecting component 11 prior to the tilting of the hollow shaft 10 is taken as C1, while the position of the base end portion 33 after the tilting is taken as C3. An angle $\beta1$ between a line segment connecting the support point O to the position C1 (or the position C3) of the base end portion 33 of the connecting component 11 and a line segment connecting the position C1 (or the position C3) to the position A1 (or the position A2) of the image pickup device 14 is a known amount ($\beta1 = \angle OC1A1 = \angle OC2A2$). In the same way, the direction $\phi$ (wherein $\phi = \angle C1A1C = \angle C2A2D$) of the visual field relative to the connecting component 11 is a known amount.

Furthermore, because there is no change in the length M1 (= the line segment C1A1 = the line segment C3A2) of the connecting component 11 after the hollow shaft 10 is tilted compared with before it was tilted, this is also a known amount.

Here, in order to expand or contract the connecting component 11 and keep the position of the optical axis of the image pickup device 14b constant after the hollow shaft 10 has been tilted, it is sufficient if the length of the connecting component 11 that corresponds to the line segment C3A2 is expanded to the optical axis n1 prior to the hollow shaft 10 being tilted. If the length of the connecting component 11 at this time is taken as M3 (= C3A3), then it is possible to calculate the Y coordinate from among the coordinates (XA3, YA3) of the intersection point A3 using each the two types of formulae given below.

[Formula 5]

$$YA3 = XA3 \times \tan(\pi - \beta) - L \times \tan(\pi - \beta)$$

[Formula 6]

$$YA3 = XA3 \times \tan(\pi - \phi - \beta) + L \times \sin\alpha + \tan(\beta - \phi - \alpha) \times L \times \cos\alpha$$

The amount of expansion or contraction of the connecting component 11 is shown by M3 - M1. The turn amount of the image pickup apparatus 14b is shown by $\alpha$. These values form a relational formula in which the tilt change amount $\alpha$ of the hollow shaft 10 is taken as the change amount. If the tilt change amount $\alpha$ of the hollow shaft 10 can be determined, then the image pickup device 14b is moved along the optical axis prior to the image pickup device 14b being tilted, and it becomes possible to obtain an image of an arbitrary point from the same direction.

Next, a method of calculating a viewing angle alteration amount is shown in typical form in FIG. 10. The viewing angle before the image pickup device 14b is tilted is taken as $\phi1$, and the focus distance before the image pickup device 14b is tilted is taken as P1. The connecting component 11 is expanded or contracted after the hollow shaft 10 has been tilted, and a position to which the image pickup device 14 is turned after this tilting in order for the image pickup device 14b prior to the hollow shaft 10 being tilted to remain constant on the optical axis is taken as A3, and the viewing angle when the observation image is adjusted so as to be the same at the position A3 is taken as $\phi3$. The focus distance where the observation image is maintained at the viewing angle $\phi3$ is taken as P3. Because the position of A3 has already been calculated, $\phi3$ and P3 are expressed in the manner shown below.

[Formula 7]

$$\phi3 = 2 \times \arctan[P1 \times \tan(\phi1/2) / P3]$$

## [Formula 8]

$$P3 = P1 - |A1A3|$$

In addition, the viewing angle alteration amount is calculated from $\phi3-\phi1$. If the position of the image pickup device 14b is controlled in the manner in accordance with the calculated viewing angle alteration amount, then even when the hollow shaft 10 is tilted using the portion that has been inserted into the body wall W1 as a support point O, the image pickup device 14b remains unaffected, and a state in which the observation image is kept constant (i.e., a state in which the observation images substantially coincide) can be maintained. Moreover, because the visual field alteration can be performed independently using the operating device 27 of the treatment tool 5, it is possible for the visual field to be altered without a user having to remove their hand from the treatment tool 5. In this manner, because the viewing point and visual field of the image pickup device 14b are manipulated by the user of the device, a scopist is not required.

In the present embodiment, in addition to the effects obtained from the first embodiment, because a device to turn the connecting component is not required, the structure of the present invention inside the body cavity can be simplified.

Here, as is shown in FIG. 6, it is also possible to provide the processor 3 with an image processing unit 3e so as to make digital zooming possible. In this case, by performing digital zooming using the image processing unit 3e so as to alter the image display range, the observation image is maintained (i.e., is kept substantially constant) after the hollow shaft 10 is tilted compared with before it was tilted. The same effects as those described above can be obtained.

Note that the present invention is not limited to the above described respective embodiments and can be broadly applied.

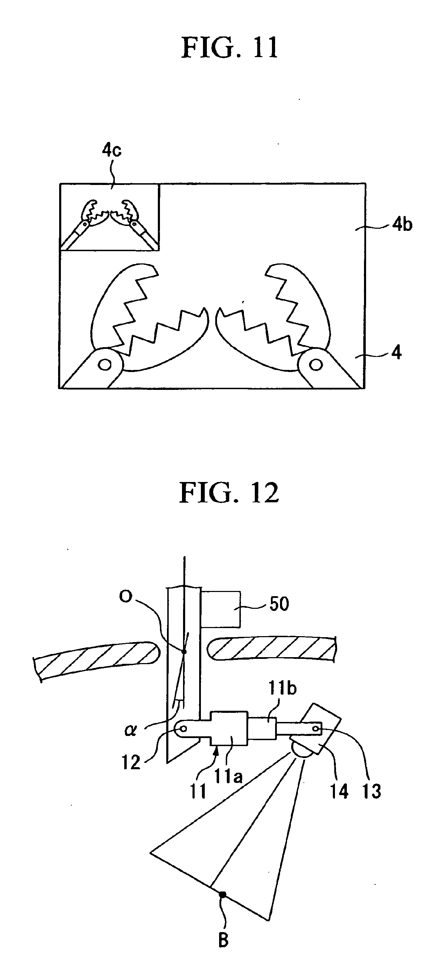

For example, as is shown in FIG 11, it is also possible to superimpose an image 4c acquired by the image pickup device 14 on an overall image 4b obtained by a rigid scope that is displayed on the display unit 4. By using the image pickup device 14 as a support for a conventional rigid scope, observation images can be obtained of different visual fields and at different magnifications. As a result, the treatment procedure is simplified.

It is also possible to use a sensor as the detection device that detects a tilt amount. Examples of such a sensor include an acceleration sensor 50 that is mounted on the hollow shaft 10 such as is shown in FIG. 12. In this case, the tilt detection unit 3d of the processor 3 shown in FIG. 1 detects an amount of change in the gravitational direction in the output from the acceleration sensor 50 that changes in accordance with the tilt of the hollow shaft 10, and thereby detects the tilt of the hollow shaft 10.

*Industrial Applicability*

The endoscopic surgery tool of the present invention can be inserted into the body cavity of a patient and used when a medical treatment procedure is being performed.

**Claims**

1. An endoscopic surgery tool (1) in which an image pickup device (14; 14a; 14b) is provided on a hollow guide component (10) that is adapted to penetrate a body wall(W1) of a living body and to guide a surgical tool(5) into a body cavity, comprising:

    a detection device (3d) that is adapted to detect an amount of change in the tilt of the guide component (10) relative to the body wall (W1); and
    a correction device (3) that is adapted to calculate from the tilt change amount an amount of movement of the image pickup device (14; 14a; 14b) that moves in conjunction with the tilting of the guide component (10), and to make corrections such that the position as well as the direction of the optical axis of the image pickup device (14; 14a; 14b) before the guide component (10) is tilted substantially coincide after it has been tilted.

2. The endoscopic surgery tool according to claim 1, wherein the correction device (3) comprises a calculation unit (3a) that calculates an amount of movement of the image pickup device such that the position as well as the direction of the optical axis of the image pickup device (14; 14a; 14b) before the guide component (10) is tilted substantially coincide after it has been tilted; and a drive device (3c) that is adapted to cause the image pickup device (14; 14a; 14b) to move in accordance with the result of the calculation by the calculation unit (3a).

**3.** The endoscopic surgery tool according to claim 2, wherein
the drive device (3c) has a connecting component (11) that has one end portion (11a) that is pivotably mounted on the guide component (10) and has another end portion (11b) that is pivotably mounted on the image pickup device (14), and in which the length from the one end portion (11a) to the other end portion (11b) can be expanded or contracted.

**4.** The endoscopic surgery tool according to claim 2, wherein
the drive device (3c) has a connecting component (11) that has one end portion (11a) that is pivotably mounted on the guide component (10) and has another end portion (11b) that is fixed to the image pickup device (14a), and in which the length from the one end portion (11a) to the other end portion (11b) can be expanded or contracted, and wherein
the correction device (3) has an image processing unit (3e) that is adapted to change the display range of images acquired by the image pickup device (14a) such that they remain substantially constant before and after the position of the image pickup device (14a) changes.

**5.** The endoscopic surgery tool according to claim 1, wherein
the correction device (3) comprises a calculation unit (3a) that calculates an amount of movement of the image pickup device (14; 14a; 14b) such that the direction of the optical axis of the image pickup device (14; 14a; 14b) remains substantially constant before and after the guide component (10) is tilted; and a drive device (3c) that is adapted to cause the image pickup device (14; 14a; 14b) to move in accordance with the result of the calculation by the calculation unit (3a), and that also is adapted to change the viewing angle of the image pickup device (14; 14a; 14b).

**6.** The endoscopic surgery tool according to claim 5, wherein
the drive device (3c) has a connecting component (11) that has one end portion (11a) that is fixed to the guide component (10) and has another end portion (11b) that is pivotably mounted on the image pickup device (14b), and in which the length from the one end portion (11a) to the other end portion (11b) can be expanded or contracted, and wherein
the correction device (3) is constructed so as to drive the connecting component (11) such that the optical axis of the image pickup device (14b) remains substantially constant before and after the image pickup device (14b) is moved in conjunction with the tilting of the guide component (10), and is constructed such that an observation image is maintained before and after the movement of the image pickup device (14b) by altering the viewing angle of the image pickup device (14b).

**7.** The endoscopic surgery tool according to claim 5, wherein
the drive device (3c) has a connecting component (11) that has one end portion (11a) that is fixed to the guide component (10) and has another end portion (11b) that is fixed to the image pickup device (14; 14a; 14b), and in which the length from the one end portion (11a) to the other end portion (11b) can be expanded or contracted, and wherein
the correction device (3) is constructed so as to drive the connecting component (11) such that the optical axis of the image pickup device (14; 14a; 14b) remains substantially constant before and after the image pickup device (14; 14a; 14b) is moved in conjunction with the tilting of the guide component (10), and is constructed such that an observation image is maintained before and after the movement of the image pickup device (14; 14a; 14b) by altering the viewing angle of the image pickup device (14; 14a; 14b) and by also altering the display range of images acquired by the image pickup device (14; 14a; 14b).

**8.** The endoscopic surgery tool according to any one of claims 2 through 7, wherein
the detection device (3d) is an acceleration sensor.

**9.** The endoscopic surgery tool according to any one of claims 2 through 7, wherein
the detection device (3d) is constructed so as to make a calculation using the amount of movement of an arbitrary point within the visual field of the image pickup device (14; 14a; 14b).

**Patentansprüche**

**1.** Endoskopisches Operationsinstrument (1), in dem eine Bildaufnahmeeinrichtung (14; 14a; 14b) an einer hohlen Führungskomponente (10) bereitgestellt ist, die dazu eingerichtet ist, eine Körperwand (W1) eines lebenden Körpers zu durchdringen und ein Operationsinstrument (5) in einen Körperhohlraum zu führen, umfassend:

eine Erfassungseinrichtung (3d), die dazu eingerichtet ist, eine Änderung in der Neigung der Führungskomponente (10) relativ zu der Körperwand (W1) zu detektieren; und

eine Korrektureinrichtung (3), die dazu eingerichtet ist, aus dem Betrag der Neigungsänderung einen Betrag der Bewegung der Bildaufnahmeeinrichtung (14; 14a; 14b), die sich in Verbindung mit der Neigung der Führungskomponente (10) bewegt, zu berechnen, und Korrekturen durchzuführen, so dass die Position und die Richtung der optischen Achse der Bildaufnahmeeinrichtung (14; 14a; 14b), bevor die Führungskomponente (10) geneigt wird und nachdem sie geneigt worden ist, im Wesentlichen zusammenfallen.

2. Endoskopisches Operationsinstrument gemäß Anspruch 1, wobei
die Korrektureinrichtung (3) eine Berechnungseinheit (3a), die einen Betrag der Bewegung der Bildaufnahmeeinrichtung berechnet, so dass die Position und die Richtung der optischen Achse der Bildaufnahmeeinrichtung (14; 14a; 14b), bevor die Führungskomponente (10) geneigt wird und nachdem sie geneigt worden ist, im Wesentlichen zusammenfallen; und ein Antriebsgerät (3c) umfasst, das dazu eingerichtet ist, die Bildaufnahmeeinrichtung (14; 14a; 14b) zu veranlassen, sich gemäß dem Ergebnis der Berechnung der Berechnungseinheit (3a) zu bewegen.

3. Endoskopisches Operationsinstrument gemäß Anspruch 2, wobei
das Antriebsgerät (3c) eine Verbindungskomponente (11) aufweist, die einen Endabschnitt (11a) hat, der schwenkbar an der Führungskomponente (10) befestigt ist, und einen weiteren Endabschnitt (11b) hat, der schwenkbar an der Bildaufnahmeeinrichtung (14) befestigt ist, und bei der die Länge von dem einen Endabschnitt (11a) zu dem anderen Endabschnitt (11b) erweitert oder verkürzt werden kann.

4. Endoskopisches Operationsinstrument gemäß Anspruch 2, wobei
das Antriebsgerät (3c) eine Verbindungskomponente (11) aufweist, die einen Endabschnitt (11a) hat, der schwenkbar an der Führungskomponente (10) befestigt ist, und einen weiteren Endabschnitt (11b) hat, der an der Bildaufnahmeeinrichtung (14a) befestigt ist, und bei der die Länge von dem einen Endabschnitt (11a) zu dem anderen Endabschnitt (11b) erweitert oder verkürzt werden kann, und wobei
die Korrektureinrichtung (3) eine Bildverarbeitungseinrichtung (3e) aufweist, die dazu eingerichtet ist, den Anzeigebereich von Bildern, die von der Bildaufnahmeeinrichtung (14a) aufgenommen wurden, so zu ändern, dass sie im Wesentlichen konstant bleiben, bevor und nachdem sich die Position der Bildaufnahmeeinrichtung (14a) geändert hat.

5. Endoskopisches Operationsinstrument gemäß Anspruch 1, wobei
die Korrektureinrichtung (3) eine Berechnungseinheit (3a), die einen Betrag der Bewegung der Bildaufnahmeeinrichtung (14; 14a; 14b) berechnet, so dass die Richtung der optischen Achse der Bildaufnahmeeinrichtung (14; 14a; 14b), bevor die Führungskomponente (10) geneigt wird und nachdem sie geneigt worden ist, im Wesentlichen konstant bleibt; und ein Antriebsgerät (3c) umfasst, das dazu eingerichtet ist, die Bildaufnahmeeinrichtung (14; 14a; 14b) zu veranlassen, sich gemäß dem Ergebnis der Berechnung der Berechnungseinheit (3a) zu bewegen, und das ferner dazu eingerichtet ist, den Blickwinkel der Bildaufnahmeeinrichtung (14; 14a; 14b) zu ändern.

6. Endoskopisches Operationsinstrument gemäß Anspruch 5, wobei
das Antriebsgerät (3c) eine Verbindungskomponente (11) aufweist, die einen Endabschnitt (11a) hat, der an der Führungskomponente (10) befestigt ist, und einen weiteren Endabschnitt (11b) hat, der schwenkbar an der Bildaufnahmeeinrichtung (14b) befestigt ist, und bei der die Länge von dem einen Endabschnitt (11a) zu dem anderen Endabschnitt (11b) erweitert oder verkürzt werden kann, und wobei
die Korrektureinrichtung (3) so konstruiert ist, dass sie die Verbindungskomponente (11) so antreibt, dass die optische Achse der Bildaufnahmeeinrichtung (14b), bevor und nachdem die Bildaufnahmeeinrichtung (14b) in Verbindung mit der Neigung der Führungskomponente (10) bewegt worden ist, im Wesentlichen konstant bleibt, und so konstruiert ist, dass ein Beobachtungsbild vor und nach der Bewegung der Bildaufnahmeeinrichtung (14b) beibehalten wird, indem der Blickwinkel der Bildaufnahmeeinrichtung (14b) geändert wird.

7. Endoskopisches Operationsinstrument gemäß Anspruch 5, wobei
das Antriebsgerät (3c) eine Verbindungskomponente (11) aufweist, die einen Endabschnitt (11a) hat, der an der Führungskomponente (10) befestigt ist, und einen weiteren Endabschnitt (11b) hat, der an der Bildaufnahmeeinrichtung (14; 14a; 14b) befestigt ist, und bei der die Länge von dem einen Endabschnitt (11a) zu dem anderen Endabschnitt (11b) erweitert oder verkürzt werden kann, und wobei
die Korrektureinrichtung (3) so konstruiert ist, dass sie die Verbindungskomponente (11) so antreibt, dass die optische Achse der Bildaufnahmeeinrichtung (14; 14a; 14b), bevor und nachdem die Bildaufnahmeeinrichtung (14; 14a; 14b) in Verbindung mit der Neigung der Führungskomponente (10) bewegt worden ist, im Wesentlichen konstant bleibt, und so konstruiert ist, dass ein Beobachtungsbild vor und nach der Bewegung der Bildaufnahmeeinrichtung (14; 14a; 14b) beibehalten wird, indem der Blickwinkel der Bildaufnahmeeinrichtung (14; 14a; 14b) geändert wird und indem ferner der Anzeigebereich von Bildern, die von der Bildaufnahmeeinrichtung (14; 14a; 14b) aufgenommen wurden, geändert wird.

**8.** Endoskopisches Operationsinstrument gemäß einem der Ansprüche 2 bis 7, wobei die Erfassungseinrichtung (3d) ein Beschleunigungssensor ist.

**9.** Endoskopisches Operationsinstrument gemäß einem der Ansprüche 2 bis 7, wobei die Erfassungseinrichtung (3d) so konstruiert ist, dass sie eine Berechnung unter Verwendung des Betrags der Bewegung eines beliebigen Punktes innerhalb des Blickfelds der Bildaufnahmeeinrichtung (14; 14a; 14b) durchführt.

**Revendications**

**1.** Outil chirurgical endoscopique (1) dans lequel un dispositif (14 ; 14a ; 14b) de capture d'images est prévu sur un composant (10) de guidage creux qui est adapté pour pénétrer une paroi (W1) de corps d'un corps vivant et pour guider un outil chirurgical (5) dans une cavité de corps, comprenant :

un dispositif (3d) de détection qui est adapté pour détecter une quantité de changement dans l'inclinaison du composant (10) de guidage par rapport à la paroi (W1) de corps ; et
un dispositif (3) de correction qui est adapté pour calculer à partir de la quantité de changement d'inclinaison une quantité de déplacement du dispositif (14 ; 14a ; 14b) de capture d'images qui se déplace en liaison avec l'inclinaison du composant (10) de guidage, et pour effectuer des corrections d'une manière telle que la position de même que la direction de l'axe optique du dispositif (14 ; 14a ; 14b) de capture d'images avant que le composant (10) de guidage soit incliné coïncident sensiblement avec après qu'il a été incliné.

**2.** Outil de chirurgical endoscopique selon la revendication 1, dans lequel le dispositif (3) de correction comprend une unité (3a) de calcul qui calcule une quantité de déplacement du dispositif de capture d'images d'une manière telle que la position de même que la direction de l'axe optique du dispositif (14 ; 14a ; 14b) de capture d'images avant que le composant (10) de guidage soit incliné coïncident sensiblement avec après qu'il a été incliné ; et un dispositif (3c) d'entraînement qui est adapté pour amener le dispositif (14 ; 14a ; 14b) de capture d'images à se déplacer conformément au résultat de calcul délivré par l'unité (3a) de calcul.

**3.** Outil de chirurgical endoscopique selon la revendication 2, dans lequel
le dispositif (3c) d'entraînement comporte un composant (11) de connexion qui possède une partie d'extrémité (11a) qui est montée de manière pivotante sur le composant (10) de guidage et possède une autre partie d'extrémité (11b) qui est montée de manière pivotante sur le dispositif (14) de capture d'images, et dans lequel la longueur allant de la partie d'extrémité (11a) à l'autre partie d'extrémité (11b) peut être allongée ou rétrécie.

**4.** Outil de chirurgical endoscopique selon la revendication 2, dans lequel
le dispositif (3c) d'entraînement comporte un composant (11) de connexion qui possède une partie d'extrémité (11a) qui est montée de manière pivotante sur le composant (10) de guidage et possède une autre partie d'extrémité (11b) qui est fixée au dispositif (14a) de capture d'images, et dans lequel la longueur allant de la partie d'extrémité (11a) à l'autre partie d'extrémité (11b) peut être allongée ou rétrécie, et dans lequel
le dispositif (3) de correction comporte une unité (3e) de traitement d'images qui est adaptée pour changer la plage d'affichage des images acquises par le dispositif (14a) de capture d'images d'une manière telle qu'elles restent sensiblement constantes avant et après le changement de la position du dispositif (14a) de capture d'images.

**5.** Outil de chirurgical endoscopique selon la revendication 1, dans lequel
le dispositif (3) de correction comprend une unité (3a) de calcul qui calcule une quantité de déplacement du dispositif (14 ; 14a ; 14b) de capture d'images d'une manière telle que la direction de l'axe optique du dispositif (14 ; 14a ; 14b) de capture d'images reste sensiblement constante avant et après l'inclinaison du composant (10) de guidage ; et un dispositif (3c) d'entraînement qui est adapté pour amener le dispositif (14 ; 14a ; 14b) de capture d'images à se déplacer conformément au résultat du calcul délivré par l'unité (3a) de calcul, et qui est également adapté pour changer l'angle de prise de vue du dispositif (14 ; 14a ; 14b) de capture d'images.

**6.** Outil de chirurgical endoscopique selon la revendication 5, dans lequel
le dispositif (3c) d'entraînement comporte un composant (11) de connexion qui possède une partie d'extrémité (11a) qui est fixée au composant (10) de guidage et possède une autre partie d'extrémité (11b) qui est montée de manière pivotante sur le dispositif (14b) de capture d'images, et dans lequel la longueur allant de la partie d'extrémité (11a) à l'autre partie d'extrémité (11b) peut être allongée ou rétrécie, et dans lequel
le dispositif (3) de correction est construit de façon à entraîner le composant (11) de connexion d'une manière telle que l'axe optique du dispositif (14b) de capture d'images reste sensiblement constant avant et après le déplacement du dispositif (14b) de capture d'images en liaison avec l'inclinaison du composant (10) de guidage, et est construit d'une manière telle qu'une image d'observation est maintenue avant et après le déplacement du dispositif (14b) de capture d'images en modifiant l'angle de prise de vue du dispositif (14b) de capture d'images.

**7.** Outil de chirurgical endoscopique selon la revendication 5, dans lequel
le dispositif (3c) d'entraînement comporte un composant (11) de connexion qui possède une partie d'extrémité (11a) qui est fixée au composant (10) de guidage et possède une autre partie d'extrémité (11b) qui est fixée au dispositif (14 ; 14a ; 14b) de capture d'images, et dans lequel la longueur allant de la partie d'extrémité (11a) à l'autre partie d'extrémité (11b) peut être allongée ou rétrécie, et dans lequel
le dispositif (3) de correction est construit de façon à entraîner le composant (11) de connexion d'une manière telle que l'axe optique du dispositif (14 ; 14a ; 14b) de capture d'images reste sensiblement constant avant et après le déplacement du dispositif (14 ; 14a ; 14b) de capture d'images en liaison avec l'inclinaison du composant (10) de guidage, et est construit d'une manière telle qu'une image d'observation est maintenue avant et après le déplacement du dispositif (14 ; 14a ; 14b) de capture d'images en modifiant l'angle de prise de vue du dispositif (14 ; 14a ; 14b) de capture d'images et également en modifiant la plage d'affichage des images acquises par le dispositif (14 ; 14a ; 14b) de capture d'images.

**8.** Outil de chirurgical endoscopique selon l'une quelconque des revendications 2 à 7, dans lequel
le dispositif (3d) de détection est un capteur d'accélération.

**9.** Outil de chirurgical endoscopique selon l'une quelconque des revendications 2 à 7, dans lequel
le dispositif (3d) de détection est construit de façon à effectuer un calcul en utilisant la quantité de déplacement d'un point arbitraire à l'intérieur du champ visuel du dispositif (14 ; 14a ; 14b) de capture d'images.

# FIG. 1

EP 1 989 990 B1

# FIG. 2

# FIG. 3

# FIG. 4

# FIG. 5

FIG. 6

# FIG. 7

EP 1 989 990 B1

# FIG. 8

EP 1 989 990 B1

# FIG. 9

# FIG. 10

# FIG. 11

# FIG. 12

**EP 1 989 990 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6648816 B